Europäisches Patentamt

European Patent Office

Office européen des brevets

(19)

(11) Numéro de publication: **0 143 717**
**A1**

(12) ## DEMANDE DE BREVET EUROPEEN

(21) Numéro de dépôt: **84402412.5**

(22) Date de dépôt: **26.11.84**

(51) Int. Cl.⁴: **A 61 B 5/02**

(30) Priorité: **25.11.83 FR 8318856**

(43) Date de publication de la demande: **05.06.85**
**Bulletin 85/23**

(84) Etats contractants désignés: **AT BE CH DE GB IT LI LU NL SE**

(71) Demandeur: **Berger, Henri, 90, Boulevard de Latour-Maubourg, F-75007 Paris (FR)**

(72) Inventeur: **Berger, Henri, 90, Boulevard de Latour-Maubourg, F-75007 Paris (FR)**
Inventeur: **Lapeyre, Didier, Chaignes, F-27120 Pacy sur Eure (FR)**

(74) Mandataire: **Gorree, Jean-Michel et al, Cabinet Plasseraud 84, rue d'Amsterdam, F-75009 Paris (FR)**

(54) **Appareil et procédé pour mesurer très rapidement le rythme cardiaque.**

(57) L'invention concerne un appareil dont le boîtier (12) a avantageusement la forme générale d'un stylo, pour mesurer par voie externe la fréquence cardiaque d'un patient, comprenant: au moins un dynamomètre (10) destiné, en fonctionnement, à être tenu en appui dans la gouttière du pouls avec une force sensiblement constante inférieure à celle créée par la pression systolique du flux sanguin de l'artère radiale; des moyens de détection des maxima du flux; des moyens de calcul pour déterminer la fréquence cardiaque; et des moyens d'affichage (11) de cette fréquence.

EP 0 143 717 A1

1

## Appareil et procédé pour mesurer très rapidement le rythme cardiaque.

La présente invention concerne un appareil et un procédé de mesure du rythme cardiaque permettant d'afficher en 2 ou 3 secondes le nombre de contractions effectuées par le coeur en une minute. Le mode opératoire classique de mesure du rythme cardiaque consiste à saisir d'une main le poignet d'un patient, à repérer au toucher l'onde pulsatile du flux sanguin de l'artère radiale, à compter zéro en prenant un repère sur le cadran d'une montre analogique ou en notant mentalement la valeur affichée par une montre à affichage digital au moment d'un maximum de l'onde pulsatile, à compter un au moment d'un second maximum de l'onde pulsatile, à compter deux au moment d'un troisième maximum de l'onde pulsatile, et ainsi de suite jusqu'à ce qu'un certain temps, en général une minute, se soit écoulé.

Un premier inconvénient de cette méthode est qu'il faut au moins une minute pour connaître avec une précision relativement bonne le rythme cardiaque d'un patient, et qu'il est nécessaire que pendant cette minute le praticien n'ait été distrait en aucune façon car cela lui aurait fait oublier soit son repère dans l'écoulement du temps soit le compte des pulsations depuis le début de son observation.

Un deuxième inconvénient de cette méthode résulte de ce que très souvent le praticien généralement pressé par le temps ne compte des passages de l'onde pulsatile que pendant quinze secondes seulement, puis qu'il multiplie par quatre le nombre de pulsations repérées depuis le début de son observation. Il en résulte une incertitude sur la détermination du rythme cardiaque d'au moins une pulsation par quinze secondes c'est-à-dire d'au moins quatre pulsations par minute. Il en résulte souvent, si en plus le praticien compte un au lieu de zéro au moment de la première pulsation repérée, une incertitude de huit pulsations par minute.

2

L'invention a essentiellement pour but de supprimer ces inconvénients dans toute la mesure du possible et de permettre à tout un chacun de mesurer sur lui-même aussi facilement que sur quelqu'un d'autre le nombre de pulsations cardiaques par minute avec une fiabilité réservée jusqu'ici à des spécialistes expérimentés opérant dans de bonnes conditions de tranquillité, et cela en un temps extrêmement court de deux ou trois secondes.

A ces fins l'invention prévoit que ledit appareil comprend :

- un capteur de force ou dynamomètre destiné à être tenu en appui dans la gouttière du pouls avec une force sensiblement constante inférieure à celle créée par la pression systolique du flux sanguin de l'artère radiale ;

- des moyens de détection connectés au susdit dynamomètre et agencés pour détecter les maxima des signaux de sortie du dynamomètre ;

- des moyens de calcul connectés aux susdits moyens de détection et agencés pour mesurer la durée du temps T séparant deux maxima des signaux de sortie du dynamomètre, calculer son inverse 1/T et multiplier celui-ci par 60, ces moyens de calcul ayant leur sortie connectée à des moyens d'affichage en vue de l'affichage, à la fin de chaque cycle cardiaque, de la fréquence ou rythme-cardiaque évaluée en nombre de pulsations par minute de temps.

L'invention sera mieux comprise à la lecture de la description détaillée qui suit de la structure et de l'utilisation d'un mode de réalisation préféré d'un appareil agencé conformément à l'invention, lequel mode de réalisation préféré n'est donné qu'à titre illustratif sans aucun caractère limitatif. Dans cette description, on se réfère aux dessins ci-annexés sur lesquels :

- La figure 1 est un graphique donnant un exemple d'évolution de la pression artérielle en fonction du temps ;

- La figure 2 est un graphique donnant des exemples des tensions fournies par le dynamomètre en fonction du temps pour 3 valeurs de la force exercée par l'utilisateur de l'appareil ;

    - La figure 3 est une vue de profil d'un mode de réalisation préféré de l'appareil suivant l'invention ;

    - La figure 4 est une vue de face de l'afficheur de l'appareil (vue suivant la flèche IV sur la figure 3) ;

    - La figure 5 donne une vue de face de la partie de l'appareil que l'utilisateur appuie dans la gouttière du pouls (vue suivant la flèche V sur la figure 3) ;

    - Et la figure 6 est un schéma-bloc illustrant la structure de l'appareil des figures 3 à 5.

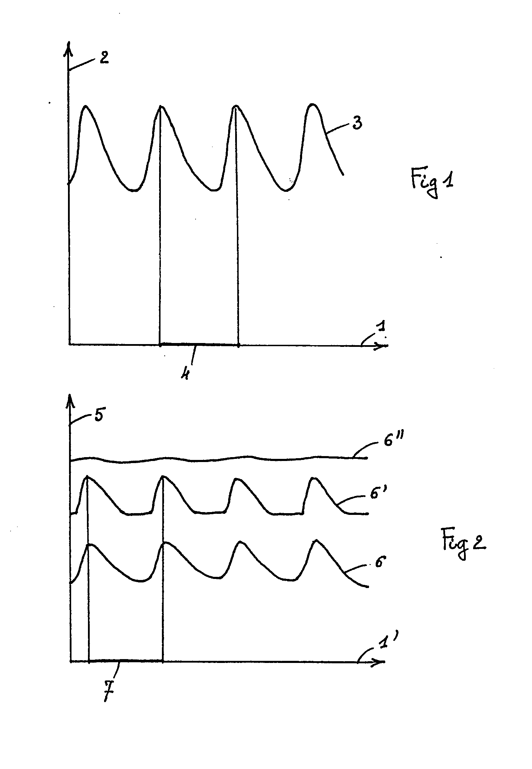

    La figure 1 représente l'évolution, en fonction du temps courant suivant l'axe 1, d'un exemple de quatre cycles de pression artérielle 3 portée suivant l'axe 2. Le segment 4 séparant deux maxima représente la grandeur T d'une période du cycle cardiaque. Les maxima correspondent à la pression systolique du flux sanguin, les minima à sa pression diastolique.

    La figure 2 représente l'évolution, en fonction du temps courant suivant l' axe 1', d'un exemple des tensions (exprimées en volts) portées suivant l'axe 5 fournies par le dynamomètre utilisé pour détecter l'onde pulsatile de la pression artérielle.

    La tension 6 est obtenue pour une force d'appui inférieure à celle créée par la pression diastolique.

    La tension 6' est obtenue pour une force d'appui supérieure à celle créée par la pression diastolique mais inférieure à celle créée par la pression systolique.

    La tension 6" est obtenue pour une force d'appui supérieure à celle créée par la pression systolique. Cette tension pratiquement constante ne permet pas de déceler les maxima de l'onde pulsatile et c'est pour cela qu'il est nécessaire que la force d'appui soit inférieure à celle créée par la pression systolique.

    On remarquera sur cette figure 2 que le segment 7 séparant deux maxima des tensions 6 ou 6' est égal au

4

segment 4 de la figure 1 c'est-à-dire à la grandeur T d'une période du cycle cardiaque.

La figure 3 représente une vue de profil d'un mode de réalisation préféré de l'appareil suivant l'invention qui a la forme d'un parallélépipède 8 aux arêtes arrondies qui peut avoir par exemple environ 8 cm de long et une section de 8 mm sur 12 mm. L'extrémité 9 est légèrement bombée et comporte une touche 10 de 5 mm de diamètre environ reliée rigidement à la membrane du dynamomètre. Celle-ci est, de préférence, en silicone conducteur et forme, avec une électrode isolée interne, une capacité variable modifiant la fréquence d'un oscillateur au même rythme que l'onde pulsatile parcourant l'artère.

L'autre extrémité 11 est constituée par un afficheur de trois caractères numériques.

Le corps 12 de l'appareil contient une ou des pile(s) électrique(s) et l'électronique nécessaire au fonctionnement. Celle-ci est sous tension en permanence dès que la ou les piles sont placées dans leur logement.

Une agrafe 13 permet la fixation de l'appareil dans la poche d'une veste ou d'une blouse à la façon d'un stylo.

La figure 4 montre l'afficheur 11 vu de face. L'affichage 000 montré ici est celui qu'il fournit avant que l'appareil ne soit appuyé sur l'artère radiale.

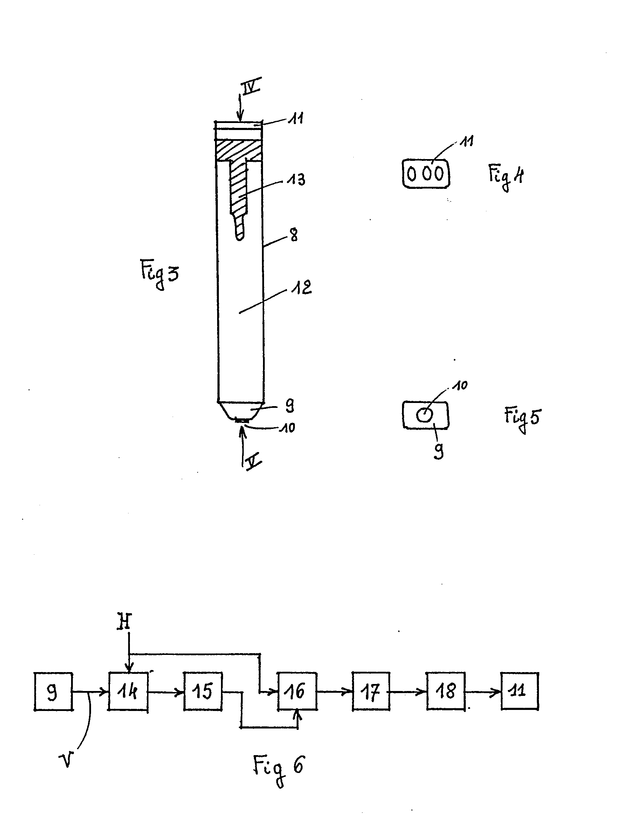

La figure 5 représente l'appareil vu de dessous montrant l'extrémité 9 contenant le dynamomètre et sa touche 10.

La figure 6 montre un exemple, sous forme de schéma-bloc, de la structure générale d'un mode de réalisation préféré de l'appareil de l'invention et de la suite des opérations effectuées sur la tension V fournie par le dynamomètre dans l'hypothèse où ce dernier donne une tension analogique continue, sous le contrôle de l'horloge H, avant de parvenir à l'afficheur 11.

A l'instant $\underline{t}$, grâce au bloc 14, la tension V est

5

échantillonnée et numérisée.

Le bloc 15 effectue la recherche des maxima de V.

Le bloc 16 accumule les incréments de l'horloge H sous le contrôle des maxima repérés par le bloc 15. Il fournit donc la période T qui est, grâce au bloc 17, inversée et multipliée par 60 pour donner le rythme cardiaque XXX qui est envoyé à la logique de commande 18 de l'afficheur 11.

Le fonctionnement de l'appareil objet de l'invention sera aisément compris par la description qui suit de son utilisation.

Lorsque l'appareil n'est pas appuyé sur l'artère radiale le signal du dynamomètre est nul et il n'y a ni maximum ni minimum ; l'afficheur indique donc 000.

Lorsque l'appareil est appuyé sur l'artère radiale dans la gouttière du pouls avec une force légère et à peu près constante comme le sont les doigts du praticien prenant le pouls, le signal du dynamomètre est automatiquement analysé c'est-à-dire qu'il y a successivement détection d'un premier maximum, déclenchement du chronomètre interne de l'appareil, détection d'un deuxième maximum, arrêt du chronomètre qui donne alors la valeur de la période T, calcul de 1/T et sa multiplication par 60, et affichage de ce résultat qui est le nombre de pulsations par minute ou rythme cardiaque.

Pour un pouls égal ou supérieur à 60 battements par minute le temps de la mesure est au maximum de deux secondes. Cette durée peut atteindre trois secondes pour un pouls compris entre 60 et 40 battements par minute.

Si l'appareil reste appuyé sur l'artère radiale, dès la détection d'un troisième maximum l'afficheur indique une nouvelle valeur du rythme cardiaque qui est évidemment identique à la précédente si le pouls est stable mais qui est différente en cas d'arythmie.

Lorsque l'appareil est écarté de l'artère radiale le dernier rythme cardiaque affiché continue à apparaître

6

pendant une minute environ, puis l'afficheur indique à nouveau 000.

Il est évident pour l'homme de l'art que le dynamomètre utilisé fournira de préférence une information digitale et que celle-ci sera avantageusement traitée par des circuits à semi-conducteur du type microprocesseur d'un usage courant aujourd'hui.

Il est également évident pour l'homme de l'art que l'appareil objet de la présente invention peut recevoir de nombreuses modifications tant dans le type de dynamomètre que dans la façon de traiter son signal, que dans la présentation ou dans la forme de l'appareil dont l'afficheur 11 ou le corps 12 peut être déporté au bout d'un cordon, émettre à distance la mesure ou être remplacé par un dispositif vocal, sans sortir du cadre de l'invention.

En conséquence, l'invention ne doit pas être interprétée comme étant limitée au mode de réalisation particulier ici décrit, elle en englobe au contraire toutes les variantes.

7

<u>REVENDICATIONS</u>

1. Appareil pour mesurer très rapidement par voie externe la fréquence cardiaque d'un patient, caractérisé en ce qu'il comprend :

- au moins un dynamomètre destiné, en fonctionnement, à être tenu en appui dans la gouttière du pouls avec une force sensiblement constante inférieure à celle créée par la pression systolique du flux sanguin de l'artère radiale,

- des moyens (15) de détection connectés au susdit dynamomètre et agencés pour détecter les maxima des signaux de sortie du dynamomètre,

- des moyens de calculs (16, 17) connectés aux susdits moyens de détection et agencés pour calculer la durée de la période T séparant deux maxima, son inverse I/T et multiplier celui-ci par 60, ces moyens de calcul ayant leur sortie connectée à

- des moyens d'affichage (11) affichant, à la fin de chaque cycle cardiaque, la fréquence cardiaque.

2. Appareil selon la revendication 1, caractérisé en ce qu'il comporte un boîtier allongé (12) présentant sensiblement l'apparence d'un stylo.

3. Appareil selon la revendication 2, caractérisé en ce que le boîtier est muni d'une agrafe (13) permettant sa fixation dans une poche.

4. Procédé pour mesurer très rapidement par voie externe la fréquence cardiaque d'un patient, caractérisé en ce qu'il comprend les étapes suivantes :

- on appuie au moins un dynamomètre dans la gouttière du pouls avec une force sensiblement constante inférieure à celle créée par la pression systolique du flux sanguin de l'artère radiale,

- on détecte deux maxima consécutifs du signal fourni par le dynamomètre,

- on mesure le temps T séparant ces deux maxima,

- on détermine l'inverse de ce temps et on multiplie par 60 cette valeur inverse, et

- on affiche la valeur du rythme cardiaque.

0143717

Pl I/2

Fig 1

Fig 2

PI II/2

Fig 3

Fig 4

Fig 5

Fig 6

Office européen
des brevets

**RAPPORT DE RECHERCHE EUROPEENNE**

0143717
Numéro de la demande

EP 84 40 2412

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 331 154 (BROADWATER et al.) <br> * Abrégé; figures 1a,b,2,5; colonne 3, ligne 34 - colonne 4, ligne 56; colonne 5, lignes 1-36; colonne 6, ligne 21 - colonne 7, ligne 66 * | 1,4 | A 61 B 5/02 |
| X | US-A-4 202 350 (WALTON) <br> * Abrégé; figures 2,4,6; colonne 2, ligne 62 - colonne 3, ligne 32; colonne 4, ligne 15 - colonne 5, ligne 28 * | 1,4 | |
| X | US-A-4 281 663 (PRINGLE) <br> * Abrégé; figures 1,2; colonne 2, lignes 25-41; colonne 4, lignes 5-34; colonne 7, lignes 45-49; colonne 9, ligne 58 - colonne 10, ligne 27 * | 1,3,4 | |
| | | | **DOMAINES TECHNIQUES RECHERCHES (Int. Cl.4)** |
| A | US-A-3 903 873 (ROYAL et al.) <br> * Abrégé; figures 6-10; colonne 3, ligne 28 - colonne 4, ligne 18; colonne 4, ligne 59 - colonne 6, ligne 16 * | 2 | A 61 B |
| A | US-A-2 572 389 (RICE) <br> * Figures 3-5; colonne 1, ligne 1 - colonne 2, ligne 35 * | 2 | |

Le présent rapport de recherche a été établi pour toutes les revendications

| Lieu de la recherche | Date d'achèvement de la recherche | Examinateur |
|---|---|---|
| LA HAYE | 27-02-1985 | CHEN A.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82